# EUROPEAN PATENT APPLICATION

(11) **EP 1 808 197 A1**
(43) Date of publication of application: **18.07.2007**
(21) Application number: 05805338.0
(22) Date of filing: 28.10.2005
(51) Int. Cl.: A61N 1/04, A61N 1/30

(54) **ELECTRODE AND IONTOPHORESIS APPARATUS**

(30) Priority: 29.10.2004 JP 2004317317; 30.06.2005 US 171953
(71) Applicant: TRANSCUTANEOUS TECHNOLOGIES INC., Shibuya-ku, Tokyo 1500022 (JP)
(72) Inventor: MATSUMURA, Akihiko, Tokyo 106-0047 (JP); MATSUMURA, Takehiko, Kanagawa, 230-0076 (JP); NAKAYAMA, Mizuo, Tokyo, 145-0062 (JP); AKIYAMA, Hidero, Tokyo, 179-0073 (JP)
(74) Representative: Schäfer, Matthias W.
(86) International application number: PCT/JP2005/019909
(87) International publication number: WO 2006/046703

(57) **Abstract**

There are provided an electrode (10a, 10b, 10c, 10d, 10e, 10f) that is capable of allowing a current to flow at a uniform current density from the entire surface of a conductive sheet (12) during the passage of a current and that solves the problem of the transfer of metal ions to a living body. The electrode (10a, 10b, 10c, 10d, 10e, 10f) comprises a conductive terminal member (11) formed of a non-metal material; and a conductive sheet (12) formed of a non-metal material and attached to the terminal member (11), the conductive sheet (12) having a specific resistance lower than a specific resistance of the terminal member (11). An Iontophoresis device (20a, 20b, 20c) and a low-frequency treatment device (50) utilizing the electrode (10a, 10b, 10c, 10d, 10e, 10f) is also disclosed.

## Description

### BACKGROUND OF THE INVENTION

### 1. Field of the Invention

The present invention relates to an electrode used for an appliance for allowing a current to flow to a living body, such as an iontophoresis device or a low-frequency treatment device. More specifically, the present invention relates to an electrode which has a low surface resistance and in which measures are taken against the transfer of metal ions to a living body. The present invention also relates to an iontophoresis device including an electrode which has a low surface resistance and in which measures are taken against the transfer of metal ions to a living body.

### 2. Description of the Related Art

An appliance such as an iontophoresis device or a low-frequency treatment device allows a current to flow to a living body (human body, etc.) through the skin so as to administer a drug or obtain the effect such as the massage.

An electrode (also called a "guide") used for allowing a current to flow to a living body in those appliances includes, in most cases, a terminal member made of a metal material for receiving a current from a device body, and a conductive sheet having a predetermined area (e.g., about 10 to 50 mmΦ, or about 10 to 50 mm per side) electrically connected to the terminal member. Furthermore, the electrode includes, in most cases, an additional member for enhancing the adhesion with respect to the skin (or for holding a drug to be administered to a living body) to be placed between the conductive sheet and the skin of the living body.

Herein, in those electrodes, in order to enhance the adhesion of an electrode with respect to a living body, and prevent the damage caused by bending and the like, a sheet material with high flexibility, such as conductive silicon rubber mixed with carbon powder or a metal thin film, is mostly used as the conductive sheet.

However, in order to enhance the flexibility of the conductive silicon rubber, it is necessary to suppress the amount of carbon to be mixed to a predetermined ratio or less. In this case, the resistance of the conductive sheet may increase.

The conductive sheet in this kind of electrode have a predetermined area so as to enhance the administration efficiency of a drug or obtain an appropriate massage effect. Therefore, it is preferable that a current be allowed to flow from the entire area of the conductive sheet. However, when the resistance of the conductive sheet increases, the current density from a site away from the terminal member on the conductive sheet decreases, with the result that a current is allowed to flow only from a slight area in the vicinity of the terminal member.

On the other hand, a conductive sheet made of a metal thin film has a low resistance in most cases, and it's flexibility can easily be made sufficient by reducing the thickness. However, while a current is allowed to flow to a living body, the metal component of the conductive sheet is ionized by electrolysis to be transferred into the living body, which may impair the health.

In this respect, it is considered that a conductive sheet made of a silver thin film has a small possibility of impairing the health. However, actually, impurity metal contained inevitably in the silver thin film is ionized to be transferred to a living body. Thus, the possibility of impairing the health cannot be eliminated completely.

### SUMMARY OF THE INVENTION

In view of the above-mentioned problems, an object of the present invention is to provide an electrode used for allowing a current to flow to a living body, which allows a current to flow at a more uniform current density from the entire surface of the conductive sheet during the passage of a current, owing to a low resistance, and which solves the problem of the transfer of metal ions to the living body, and an iontophoresis device using the electrode.

The above-mentioned problems has been overcome by an electrode including a conductive terminal member formed of a non-metal material; and a conductive sheet formed of a non-metal material and attached to the terminal member, in which the conductive sheet has a specific resistance lower than a specific resistance of the terminal member.

That is, according to the present invention, both of the terminal member for receiving a current from an appliance such as an iontophoresis device or a low-frequency treatment device, and the conductive sheet for allowing a current to flow to a living body are made of a material containing no metal. Therefore, the problem of the transfer of metal ions to a living body during the passage of a current can be eliminated.

Further, the conductive sheet and the terminal member are provided as separate members that are both formed of a non-metal material. Hence, the material for the conductive sheet having a low specific resistance can be selected from a wide variety of non-metal materials, as long as the material can attain a sufficient security level for the living body and has a certain level of flexibility. Meanwhile, the terminal member can be made of a material having even a little high specific resistance as longas the terminal can secure the requisite strength, durability, and chemical resistance. In this way, it is possible to expand the range of choices for materials.

In this case, the conductive sheet preferably has a surface resistance of 1 to 30 Ω/(square), particularly preferably 1 to 10 Ω/(square). This allows current to flow at a substantially uniform current density from the entire surface of the conductive sheet.

As a specific structural example that attains sufficient flexibility appropriate for the use for a living body and the above-described surface resistance, the conductive sheet of the present invention is preferably made of carbon fibers or carbon fiber paper.

As regards the carbon fibers, as long as the carbon fibers have sufficiently high conductivity to allow a current to flow at a substantially uniform current density from the entire surface of the conductive sheet, any kinds of carbon fibers, such as natural fiber hydrocarbon, polyacrylonitrile carbon fibers, pitch carbon fibers, and rayon carbon fibers, can be used. As regards the carbon fiber paper, any carbon fiber paper obtained by molding carbon fibers into a mat shape or a paper shape by a paper making technique can be used as the carbon fiber paper.

The conductive sheet of the present invention can be formed of carbon fibers or carbon fiber paper impregnated with a polymer elastomer as well. This prevents quality deterioration of the electrode that results from peeled carbon fibers or carbon fiber paper, and facilitates the handling of the electrode during the manufacturing process.

Note that the polymer elastomer used herein is preferably a material having high flexibility and containing no toxic substance such as thermoplastic polyurethane or silicon rubber.

In addition, the polymer elastomer is preferably imparted with a certain level of conductivity, for example, by dispersing a non-metal filler into the polymer elastomer, with the aim of reducing a contact resistance between the carbon fibers or carbon fiber paper, and the skin.

The terminal member of the present invention preferably includes the polymer matrix and non-metal conductive filler dispersed in the polymer matrix.

Inthiscase, silicon rubber or silicon resin is particularly preferably used as the polymer matrix in terms of the safety to a living body. However, a rubber material containing other natural rubber and synthetic rubber, or a synthetic resin material containing a thermosetting resin and thermoplastic resin can also be used, as long as it can provide the terminal member with characteristics such as mechanical strength and durability sufficient for playing a role as a connection terminal.

As the non-metal filler mixed in the high-molecular-weight matrix, carbon is particularly preferred. Specific examples thereof include graphite, black lead, carbon black, fine powder of glass-shaped carbon, and short fibers obtained by cutting carbon fibers.

The amount of carbon to be mixed with the polymer matrix can be determined in conjunction with the strength and conductivity required for the terminal member. As is apparent from an embodiment of the present invention described later, the terminal member of the present invention can be configured so as to have a relatively large cross-section and a small length. Therefore, it is not necessarily required that the terminal member of the present invention have a corn position with high conductivity. Forexample, in the case of using silicon rubber as the polymer matrix and carbon black as the non-metal filler, the terminal member can have a composition in which 20 to 60 parts by weight of carbon black are mixed with respect to 100 parts by weight of silicon rubber.

In the electrode of the present invention, a part of the polymer matrix constituting the terminal member, or a part of the polymer matrix and a part of the non-metal filler are solidified under the condition of being impregnated with carbon fibers or carbon fiber paper, whereby the terminal member can be attached to a conductive sheet. In this case, it is not necessary to provide a member for attaching the terminal member projecting to a front side (living body side) of the conductive sheet, so that the problem of a decrease in adhesion between the skin and the electrode, which occurs in the case of providing a projection part on the front side of the conductive sheet, can be eliminated.

According to the present invention, the terminal member can be attached to the conductive sheet by integral molding, which can reduce the production cost of the electrode.

Furthermore, the terminal member in the present invention can be provided with a male (or female) fitting portion to be fitted in a female (or male) fitting portion of a connector to be connected to a power source of an iontophoresis device, a low-frequency treatment device, or the like. This can enhance the convenience of a connection operation.

Furthermore, the electrode of the present invention can be particularly preferably used in an iontophoresis device in which it is desired to allow a current to flow at a uniform current density from a larger area so as to obtain higher administration efficiency of a drug with a lower voltage, and it is necessary to avoid the transfer of metal ions to a living body.

In this case, it is preferable to use the electrode of the present invention in at least one of a working electrode structure and a nonworking electrode structure provided in the iontophoresis device. For example, in the case of an iontophoresis device for administering a drug that is dissociated to minus ions, the electrode of the present invention is preferably used at least in the nonworking electrode structure. In the case of an iontophoresis device for administering a drug that is dissociated to plus ions, the electrode of the present invention is preferably used at least in the working electrode structure.

Furthermore, the iontophoresis device of the present invention includes a power source, a working electrode structure, and a nonworking electrode structure. The working electrode structure includes: a first electrode connected to a terminal of a first conductivity of the power source; a first conductive medium layer placed on a front side of the first electrode; a first ion-exchange membrane for selecting ions of a second conductivity that is opposite to the first conductivity, the first ion-exchange membrane being placed on a front side of the first conductive medium layer; a drug layer for holding a drug solution containing a drug that is dissociated to ions of the first conductivity, the drug layer being placed on a front side of the first ion-exchange membrane; and a second ion-exchange membrane for selecting ions of the first conductivity, the second ion-exchange membrane being placed on a front side of the drug layer. The nonworking electrode structure includes a second electrode connected to a terminal of the second conductivity of the power source and a second conductive medium layer placed on a front side of the second electrode. It is particularly preferable that at least one of the first electrode and the second electrode includes a conductive terminal member formed of a non-metal material and a conductive sheet formed of a non-metal material attached to the terminal member, and the conductive sheet has a specific resistance lower than a specific resistance of the terminal member. Because of this, an iontophoresis device canbe realized, which has the effects of efficiently administering drug ions to a living body by suppressing the transfer of ions having a conductivity opposite to that of drug ions from the living body to the working electrode, and preventing the adverse influence on the skin of the living body caused when H⁺ ions, OH⁻ ions, and the like generated in the vicinity of the conductive sheet of the working electrode structure are transferred to the drug layer to change a pH, and in addition, which is capable of administering the drug ions to the living body efficiently at a uniform current density from the entire area of the conductive sheet without the possibility of the transfer of metal ions to the living body.

Furthermore, the nonworking electrode structure in the above-mentioned iontophoresis device can further include a third ion-exchange membrane for selecting ions of the second conductivity, the third ion-exchange membrane being placed on a front side of the second conductive medium layer, or can include a fourth ion-exchange membrane for selecting ions of the first conductivity, the fourth ion-exchange membrane being placed on a front side of the second conductive medium layer, a third conductive medium layer placed on a front side of the fourth ion-exchange membrane, and a fifth ion-exchange membrane for selecting ions of the second conductivity, the fifth ion-exchange membrane being placed on a front side of the third conductive medium layer. In this case, the following problems can be reduced: the increase in resistance of the passage of a current caused by oxygen gas, chlorine gas, and the like generated by electrolysis in the conductive medium layer of the non working electrode structure, the adverse influence of toxic gas such as chlorine gas on the living body, the damage to the skin of the living body caused by the change in pH due to H⁺ ions and OH- ions generated in the vicinity of the conductive sheet of the nonworking electrode structure. Thus, a drug can be administered stably under the condition of the stable passage of a current for a long period of time.

In the above-mentioned structures of the present invention, the first or second conductivity refers to a plus or a minus. The ion-exchange membrane for selecting ions of the first or second conductivity refers to an ion-exchange membrane for selecting plus ions or minus ions, i.e., a cation exchange membrane or an anion exchange membrane.

### BREIF DESCRIPTION OF THE DRAWINGS

In the accompanying drawings:
FIG. 1A is a plan view of an electrode according to one embodiment of the present invention, and FIGS. 1B and 1C are cross-sectional views of the electrode;
FIGS. 2A to 2C are cross-sectional views of an electrode according to another embodiment of the present invention;
FIGS. 3A and 3B are cross-sectional views of an electrode of still another embodiment of the present invention;
FIG. 4 illustrates a structure of an iontophoresis device according to one embodiment of the present invention, using the electrode of the present invention;
FIG. 5 illustrates a structure of an iontophoresis device according to another embodiment of the present invention;
FIG. 6 illustrates a structure of an iontophoresis device according to still another embodiment of the present invention; and
FIG. 7 illustrates a use embodiment of the electrode of the present invention in a low-frequency treatment device.

### DESCRIPTION OF THE PREFERRED EMBODIMENTS

Hereinafter, an embodiment of the present invention will be described with reference to the drawings.

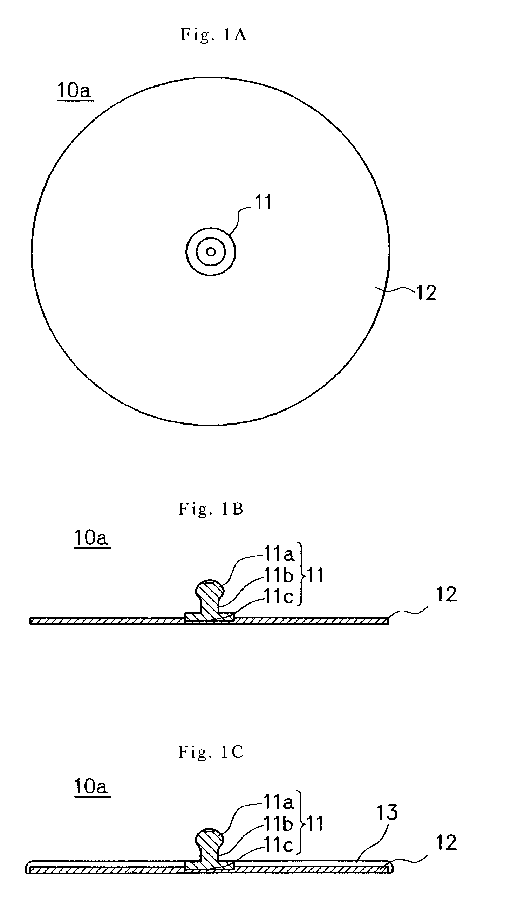

FIG. 1A is a plan view of an electrode 10a according to one embodiment of the present invention. FIGS. 1B and 1C are cross-sectional views of the electrode 10a.

As shown in FIGS. 1A and 1B, the electrode 10a includes: a terminal member 11 formed of conductive silicon rubber including a male fitting portion 11a, a body portion 11b, and a junction portion 11c; and a conductive sheet 12 made of carbon fibers obtained by carbonating woven fabric such as silk or cotton by a high-temperature treatment.

The terminal member 11 is obtained by vulcanizing a compound in which 50 parts by weight of carbon black and 5 parts by weight of sulfur-based vulcanizing agent with 100 parts by weight of silicon rubber at 140 to 160°C in a mold placed on the conductive sheet 12. Silicon rubber and carbon black in the compound are solidified under the condition of that the silicon rubber and the carbon black are impregnated in the carbon fibers constituting the conductive sheet 12 during a vulcanizing treatment, whereby the terminal member is integrated with the conductive sheet 12.

The electrode 10a can be provided with a cover 13 as shown in FIG. 1C so that the upper surface of the conductive sheet 12 is protected, or in the case where the electrode 10 a is combined with a liquid such as a conductive medium as described later, the liquid is prevented from exuding to an upper part of the conductive sheet 12.

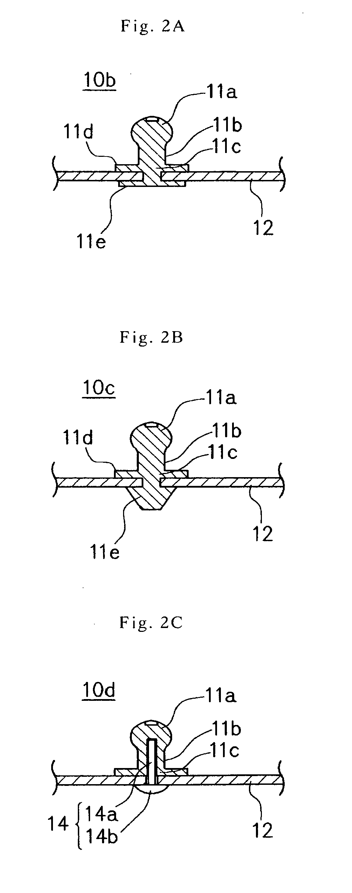

FIGS. 2A to 2C are cross-sectional views of electrodes 10b to 10d according to another embodiment of the present invention.

The electrodes 10b to 10d in FIGS. 2A to 2C each include the terminal member 11 and the conductive sheet 12 made of the same materials as those of the electrode 10a. However, the electrodes 10b to 10d are different from the electrode 10a shown in FIG. 1 in the way of junctioning the conductive sheet 12 with respect to the terminal member 11.

In the electrode 10b shown in FIG. 2A, engagement portions 11d and 11e are formed at a lower part of the terminal member 11. The conductive sheet 12 is attached to the terminal member 11 by inserting the engagement portion 11e in a small hole provided at the center of the conductive sheet 12. In the electrode 10c in FIG. 2B, by reducing the width of the engagement portion 11e and tapering the engagement portion 11e, the engagement portion 11e can be easily inserted in the small hole of the conductive sheet 12. Furthermore, in the electrode 10d in FIG. 2C, an axial hole is formed in the body portion 11b of the terminal member 11, and an axis 14a of a stopper 14 formed of conductive silicon rubber similar to the material of the terminal member 11 is embedded in the axial hole, whereby the conductive sheet 12 is clipped by the engagement portion 14b of the stopper 14.

In each of the electrodes 10a to 10d, wiring from an appliance such as an iontophoresis device or a low-frequency treatment device is connected to the male fitting portion 11a, and a current to a living body is guided to the skin of the living body placed below the conductive sheet 12 through the male fitting portion 11a, the body portion 11b, the junction portion 11c and the conductive sheet 12.

In this case, the dimensions of the body portion 11b can be set so as to have a large diameter (e.g., 1 to 3 mmΦ) and a small length (0.5 to 2 mm) . Therefore, even in the case where the material constituting the terminal member 11 does not have high conductivity, it is easy to prevent the passage of a current to the conductive sheet 12 from being hindered, by appropriately designing the shape and dimensions of the terminal member 11. Thus, in the selection of the material for the terminal member 11, the priority can be given to the characteristics such as the strength, durability, and chemical resistance.

The conductive sheet made of carbon fibers has a very low surface resistance, for example, 1 to 10 Ω/(square) (4-probe method defined in JIS K7 194). Therefore, a current guided from the junction portion 11c is guided to the skin of a living body uniformly from the entire area of the conductive sheet.

Note that any carbon fiber papers which is made by molding carbon fibers into a mat shape or a paper shape by a paper making technique can also be used for the conductive sheet 12 of the electrodes 10a to 10d in place of the carbon fibers. Alternatively, it is possible to use the carbon fibers and carbon fiber paper impregnated with a polymer elastomer such as thermoplastic polyurethane or silicon rubber. In the case as well, the surface resistance of the conductive sheet can be set to a value as low as 1 to 10 Ω/(square).

Furthermore, a metal material is not used in the electrodes 10a to 10d, so that there is no possibility that ionized metal is transferred to a living body.

Furthermore, as described later, depending upon the use purpose of the electrode of the present invention, under the condition that a thin film member impregnated with a conductive medium is interposed between the conductive sheet 12 and the skin of a living body or under the condition that the conductive sheet 12 is soaked with a conductive medium, a current may be allowed to flow to the living body. In each of the electrodes 10a to 10d, a part of the conductive medium permeates the carbon fibers of the conductive sheet 12, and the conducting state between the conductive sheet 12 and the thin film member, or that between the conductive sheet 12 and the conductive medium can be made satisfactory.

Furthermore, the passage of a current from an appliance such as an iontophoresis device or a low-frequency treatment device may be performed by connecting a connector made of metal having a female fitting portion to be fitted to the male fitting portion 11a. In each of the electrodes 10a to 10d of the present invention, the male fitting portion 11a, which may come into contact with a member made of metal, and the conductive sheet 12 are separated by the body portion 11b. Or in the case where the cover 13 is provided on the conductive sheet 12, the conductive sheet 12 is further protected by the cover 13. Therefore, the generation of metal ions due to the electrolysis of the member made of metal, and the transfer of such metal ions to the conductive sheet 12 or the conductive medium are prevented.

As described above, any of the electrodes 10a to 10d have characteristics preferable as those which are used for allowing a current to flow to a living body. Among them, the electrode 10a has a structure without a convex projection on the side of the conductive sheet 12, unlike the engagement portions 11e and 14b in the electrodes 10b to 10d. Thus, the electrode 10a is particularly preferable in terms of keeping the adhesion state more satisfactory between the skin of a living body and the electrode.

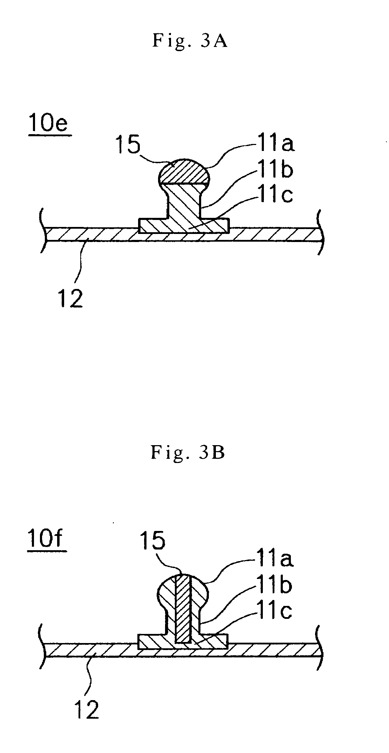

In addition to the above-mentioned structure, as illustrated in 10e and 10f in FIGS. 3A and 3B, a reinforcing member 15 made of metal can also be attached to the terminal member 11 in the electrode of the present invention. This can enhance the strength and durability of the terminal member 11, or make the contact satisfactory between the terminal member 11 and the connector in the case of allowing a current to flow to the electrode via the connector.

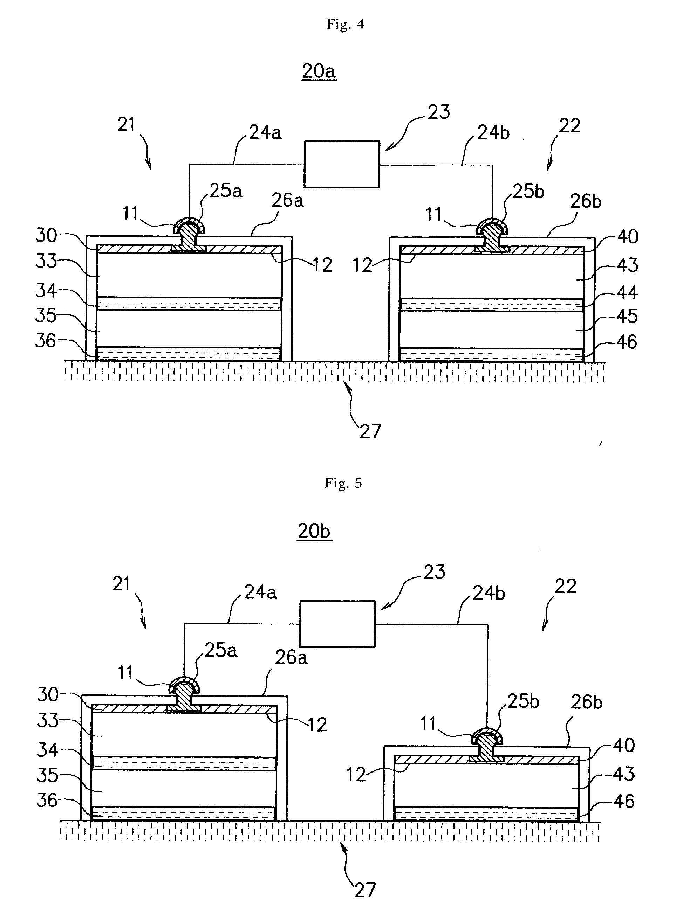

FIG. 4 is an explanatory view showing a structure of an iontophoresis device using the electrode of the presentinvention.

As shown in the figure, the iontophoresis device 20a includes, as main components (members), a working electrode structure 21, a nonworking electrode structure 22, and a power source 23. Reference numeral 27 denotes the skin (or the membrane) of a living body.

The working electrode structure 21 includes: an electrode 30 connected to a terminal of a first conductivity of the power source 23 via a cord 24a and a female connector 25a; a first conductive medium layer 33 placed so as to be electrically connected to the electrode 30; an ion-exchange membrane 34 for selecting ions of a conductivity opposite to the first conductivity (hereinafter, referred to as a "second conductivity"), the ion-exchange membrane being placed on a front side of the first conductive medium layer 33; a drug layer 35 placed on a front side of the ion-exchange membrane 34; and an ion-exchange membrane 36 for selecting ions of the first conductivity, the ion-exchange membrane being placed on a front side of the drug layer 35, and the entire laminate is housed in a cover or a container 26a.

Furthermore, the nonworking electrode structure 22 includes: an electrode 40 connected to a terminal of the second conductivity of the power source 23 via a cord 24b and a female connector 25b; a second conductive medium layer 43 placed so as to be electrically connected to the electrode 40; anion-exchange membrane 44 for selecting ions of the first conductivity, the ion-exchange membrane being placed on a front side of the second conductive medium layer 43; a third conductive medium layer 45 placed on a front side of the ion-exchange membrane 44; and an ion-exchange membrane 46 for selecting ions of the second conductivity, the ion-exchange membrane being placed on a front side of the third conductive medium layer 45, and the entire laminate is housed in a cover or a container 26b.

Herein, the electrodes 30 and 40 each include: a terminal member 11 formed of conductive silicon rubber including a male fitting portion 11a, a body portion 11b, and a junction portion 11c; and a conductive sheet 12 made of carbon fibers obtained by carbonizing woven fabric such as silk or cotton by a high-temperature treatment, in the same way as in the electrodes 10a to 10f shown in FIGS. 1 and 2.

In this case, the shapes and dimensions of the terminal member 11 and the conductive sheet 12 can be determined appropriately inconsideration of the strength and handleability of the electrodes 30 and 40, the administration efficiency of a drug, and the like. As an example, the terminal member 11 may have a composition in which 20 to 60 parts by weight of carbon black is compounded with respect to 100 parts by weight of silicon rubber; the male fitting portion 11a may be formed in a curved shape of about 2.3 mmΦ; the body portion 11b may be formed in a cylinder shape of 2.0 mmΦ with a length of about 10 mm; the junction portion 11c may be formed in a disk shape of about 4.0mmΦ with a thickness of about 0.5 mm; and the conductive sheet 12 may be formed in a circular sheet of 3 mmΦ (thickness: about 0.5 mm) made of carbon fibers obtained by carbonizing woven fabric such as silk or cotton by a high-temperature treatment.

A conductive medium such as phosphate buffered saline or physiological saline is used as each of the conductive medium layers 33, 43, and 45 in order to make the conduction with respect to the conductive sheet 12 of the electrode 30 satisfactory.

Furthermore, in order to prevent the generation of gas and the change in pH caused by the elecrolysis of a conductive medium occurring in the vicinity of a contact portion with respect to the conductive sheet 12, a compound that is more easily to be oxidized or reduced than the electrolysis of water (the oxidation at a positive electrode and the reduction at a negative electrode) can be added to the above-mentioned conductive medium. In terms of the biological safety and economical efficiency (low cost and ease of availability), for example, an inorganic compound such as ferrous sulfate or ferric sulfate, a medical agent such as ascorbic acid (vitamin C) or sodium ascorbate, an acid compound present on the skin surface such as lactic acid, or an organic acid such as oxalic acid, malic acid, succinic acid, or fumaric acid and/or a salt thereof can be usedpreferably. Those compounds can be added alone or in combination.

Furthermore, each of the conductive medium layers 33, 43, and 45 may hold the above-mentioned conductive medium in a liquid state. Alternatively, in order to enhance the handleability, each of the conductive medium layers 33, 43, and 45 may hold a water-absorbing thin film formed of a polymer material or the like impregnated with the above-mentioned conductive medium.

An acrylic hydrogel film, a segmented polyurethane gel film, an ion-conductive porous sheet for forming a gel solid electrolyte (e.g., porous polymer based on an acrylonitrile copolymer with a porosity of 20 to 80% containing 50 mol% or more of acrylonitrile (preferably 70 to 98 mol%), disclosed by JP 11-273452 A), or the like can be used as the material for the water-absorbing thin film. The impregnation ratio (100 × (W-D)/D[%], where D is a weight in a dry state and W is a weight after impregnation) of the conductive medium to be impregnated in the thin film is preferably 30 to 40%.

The drug layer 35 holds a solution of a drug dissociated to ions of the first conductivity that is the same as that of the terminal to which the working electrode structure 21 is connected.

The drug layer 35 may also hold a drug solution in a liquid state in the same way as in the conductive medium layers 33, 43, and 45. Alternatively, in order to enhance the handleability and the like, the drug layer 35 may hold a water-absorbing thin film formed of a polymer material or the like (e.g., an acrylic hydrogel film) impregnated with a drug solution.

As the ion-exchange membranes 34, 36, 44, and 46 for selecting ions of the first or second conductivity, a cation exchange membrane such as NEOSEPTA, CM-1, CM-2, CMX, CMS, or CMB produced by Tokuyama Co. , Ltd., or an anion exchange membrane such as NEOSEPTA, AM-1 , AM-3, AMX, AHA, ACH, or ACS produced by Tokuyama Co., Ltd. can be used. In particular, a cation exchange membrane in which a part or an entirety of a pore of a porous film is filled with an ion-exchange resin having a cation exchange function, or an anion exchange membrane filled with an ion-exchange resin having an anion exchange function can be used preferably.

Herein, a fluorine type resin with an ion-exchange group introduced to a perfluorocarbon skeleton or a hydrocarbon type resin containing a resin that is not fluorinated as a skeleton can be used as the above-mentioned ion-exchange resin. In view of the convenience of a production process, a hydrocarbon type ion-exchange resin is preferable. Although the filling ratio of the ion-exchange resin is also related to the porosity of the porous film, the filling ratio is generally 5 to 95% by mass, in particular, 10 to 90% by mass, and preferably 20 to 60% by mass.

There is no particular limit to an ion-exchange group of the above-mentioned ion-exchange resin, as long as it is a functional group generating a group having negative or positive charge in an aqueous solution. As specific examples of the functional group to be such an ion-exchange group, those of a cation exchange group include a sulfonic acid group, a carboxylic acid group, and a phosphonic acid group. Those acid groups may be present in the form of a free acid or a salt. Examples of a counter cation in the case of a salt include alkaline metal cations such as sodium ions and potassium ions, and ammonium ions. Of those cation exchange groups, generally, a sulfonic acid group that is a strong acidic group is particularly preferable. Examples of the anion exchange group include primary to tertiary amino groups, a quaternary ammonium group, a pyridyl group, an imidazole group, a quaternary pyridinium group, and a quaternary imidazolium group. Examples of a counter anion in those anion exchange groups include halogen ions such as chlorine ions and hydroxy ions. Of those anion exchange groups, generally, a quaternary ammonium group and a quaternary pyridinium group that are strong basic groups are used preferably.

A film shaped or a sheet shaped sheet having a number of small holes communicating the front surface and the back surface thereof is used as the above-mentioned porous film without any particular limit. In order to satisfy both the high strength and the flexibility, it is preferable that the porous film be made of a thermoplastic resin.

Examples of the thermoplastic resins constituting the porous film include, without limitation: polyolefin resins such as homopolymers or copolymers of α-olefins such as ethylene, propylene, 1-butene, 1-pentene, 1-hexene, 3-methyl-1-butene, 4-methyl-1-pentene, and 5-methyl-1-heptene; vinyl chloride resins such as polyvinyl chloride, vinyl chloride-vinyl acetate copolymers, vinyl chloride-vinylidene chloride copolymers, and vinyl chloride-olefin copolymers; fluorine resins such as polytetrafluoroethylene, polychlorotrifluoroethylene, polyvinylidene fluoride, tetrafluoroethylene-hexafluoropropylene copolymers, tetrafluoroethylene-perfluoroalkyl vinylether copolymers, and tetrafluoroethylene-ethylene copolymers; polyamide resins such as nylon 6 and nylon 66; and those which are made from polyimide resins. Polyolefinresins are preferably used as they are superior in mechanical strength, flexibility, chemical stability, and chemical resistance, and have good compatibility withion-exchange resins. As the polyolefin resins, polyethylene and polypropylene are particularly preferable and polyethylene is most preferable.

There is no particular limit to the property of the above-mentioned porous film made of the thermoplastic resin. However, the average pore diameter of pores may be preferably 0.005 to 5.0 µm, more preferably 0.01 to 2.0 µm, and most preferably 0.02 to 0.2 µm since the porous film having such an average pore diameter is likely to be a thin ion-exchange membrane having excellent strength and a low electric resistance. The average pore diameter in the present specification refers to an average flow pore diameter measured in accordance with a bubble point method (JIS K3832-1990). Similarly, the porosity of the porous film may be preferably 20 to 95%, more preferably 30 to 90%, and most preferably 30 to 60%. Furthermore, the thickness of the porous film may be preferably 5 to 140 µm, more preferably 10 to 120 µm, and most preferably 15 to 55 µm. Usually, an anion exchange membrane or a cation exchange membrane using such a porous film has a thickness of the porous film with +0 to 20 µm.

In the above-mentioned iontophoresis device 20a, the drug in the drug layer 35 dissociated to ions of the first conductivity is administered to a living body via the ion-exchange membrane 36 and the skin 27 with a voltage applied from the power source 23.

In this case, owing to the function of the ion-exchange membranes 34, 36, 44, and 46, ions of a conductivity opposite to that of the drug ions are prevented from being transferred from the living body side to the drug layer 35 side, and H⁺ and OH⁻ generated at the electrodes 30 and 40 are suppressed from moving to the skin 27 side, whereby drug ions can be administered stably with satisfactory efficiency for a long period of time while the change in pH on the skin 27 is suppressed.

Furthermore, in the iontophoresis device 20a, the conductive sheet 12 of each of the electrodes 30 and 40 is made of carbon fibers with a low resistance. Therefore, a current is allowed to flow through the conductive medium layer 33/ion-exchange membrane 34/drug layer 35/ion-exchange membrane 36, or the conductive medium layer 43/ion-exchange membrane 44/conductive medium layer 45/ion-exchange membrane 46 at a very uniform current density from the entire surface of the conductive sheet 12.

Thus, the administration efficiency of a drug to a living body is higher in the iontophoresis device of the present invention, compared with the conventional iontophoresis device in which a current is allowed to flow in a state where the current is concentrated in a narrow area in the vicinity of the terminal member owing to the use of the conductive sheet formed of conductive silicon rubber having a high electric resistance.

Furthermore, unlike the conventional iontophoresis device using a conductive sheet made of a thin film of metal such as silver, in the iontophoresis device of the present invention, it is not necessary to use a metal material in the working electrode structure 21 and the nonworking electrode structure 22. Therefore, the transfer of metal ions generated by electrolysis or the like to a living body is prevented.

Furthermore, in the case where the conductive medium layers 33 and 43 each hold a conductive medium in a liquid state, or in the case where the conductive medium layers 33 and 43 each holds a water-absorbing thin film formed of a polymer material or the like impregnated with a conductive medium, a part of the conductive medium permeates the carbon fibers constituting the conductive sheet 12 of each of the electrodes 30 and 40, depending upon the impregnation amount, and the conducting state between the conductive sheet 12 and the conductive medium layers 33 and 43 can be kept more satisfactorily.

On the other hand, the conductive sheet 12 and the female connectors 25a and 25b are partitioned at least by the body portion 11b. Therefore, even in the case where the female connectors 25 and 25b are made of metal, and even in the case where the conductive medium of each of the conductive medium layers 33 and 43 permeates the conductive sheet 12, there is no possibility that the metal component of each of the female connectors 25a and 25b is ionized to be transferred to the conductive sheet 12, or is transferred further to a living body.

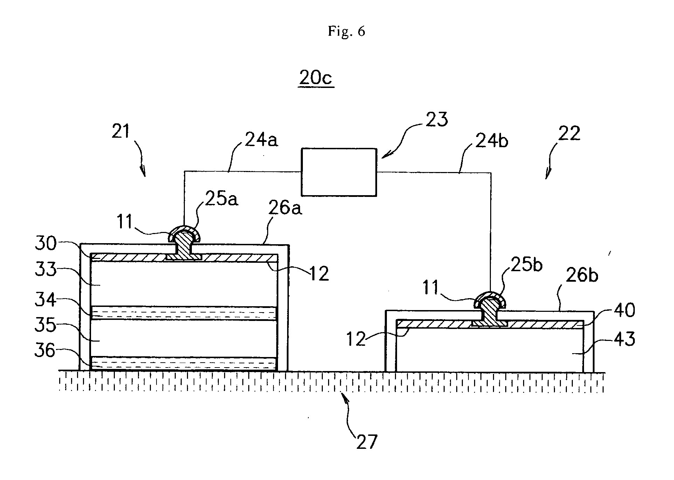

FIGS. 5 and 6 illustrate structures of iontophoresis devices 20b and 20c according to other embodiments.

The iontophoresis device 20b has the same structure as that of the iontophoresis device 20a shown in FIG. 4, except that the nonworking electrode structure 22 does not have the ion-exchange membrane 44 and the third conductive medium layer 45. The iontophoresis device 20c has the same structure as that of the iontophoresis device 20a shown in FIG. 4, except that the nonworking electrode structure 22 does not have the ion-exchange membrane 44, the third conductive medium layer 45, and the ion-exchange membrane 46. Although the iontophoresis devices 20b and 20c cannot achieve the performance of suppressing the change in pH on a contact surface of the nonworking electrode structure 22 with respect to the skin 27, comparable to that of the iontophoresis device 20a, the iontophoresis devices 20b and 20c exhibit the same performance as that of the iontophoresis device 20a in the other aspects. In particular, in the same way as in the iontophoresis device 20a, the iontophoresis devices 20b and 20c exhibit the effects such as the enhancement of the administration efficiency of a drug due to the passage of a current at a uniform current density from the entire surface of the conductive sheet 12, the elimination of the possibility of the transfer of metal ion stoa living body, and the maintenance of the sat is factory conducting state between the conductive sheet 12 and each of the conductive medium layers 33 and 43.

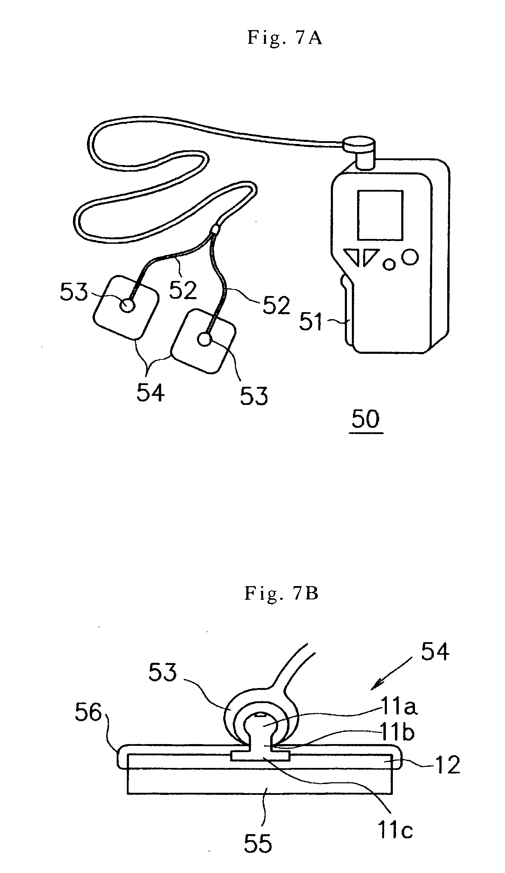

FIG. 7 illustrates the use embodiment of the electrode of the present invention in a low-frequency treatment device 50.

As shown in FIG. 7, the low-frequency treatment device 50 includes alow-frequency treatment body 51, and a set of electrodes 54 and 54 receiving a current via cords 52 and 52 and female connectors 53 and 53 from the low-frequency therapeutic body 51.

In the same way as in the electrodes 10a to 10f shown in FIGS. 1 and 2, the electrode 54 includes: a terminal member 11 formed of conductive silicon rubber including a male fitting portion 11a, a body portion 11b, and a junction portion 11c; and a conductive sheet 12 formed of carbon fibers obtained by carbonizing woven fabric such as silk or cotton by a high-temperature treatment.

Furthermore, a conductive adhesive layer 55 made of a gel such as polyhydroxymethacrylate impregnated with a conductive medium such as a potassium chloride aqueous solution is placed below the conductive sheet 12. A current is allowed to flow to the skin via the conductive adhesive layer 55.

In the figure, reference numeral 56 denotes a cover for protecting the upper surface of the conductive sheet 12.

In the electrodes 54 and 54, the conductive sheet 12 formed of carbon fibers having a low resistance is used, so that a current is allowed to flow at a very uniform current density from the entire surface of the conductive sheet 12. Thus, the function such as the massage can be performed with respect to a living body without giving discomfort caused when a currentis concentrated in a narrow range.

Furthermore, in the low-frequency treatment device 50, it is not necessary to use metal members for the electrode 54, the conductive adhesive layer 55, and the like, so that there is no possibility that metal ions are transferred to a living body during the passage of a current.

Generally, a metal member is used as the female connector 53. In the low-frequency treatment device 50, at least the body portion 11b is interposed between the female connector 53 and the conductive sheet 12, so that the metal component of the female connector 53 is prevented from being ionized to be transferred to the conductive adhesive layer 55, and further to a living body.

Although the present invention has been described by way of some embodiments, the present invention is not limited to these embodiments, and can be variously modified within the scope of the claims.

For example, in addition to silicon rubber, examples of the polymer matrix that may be used in the terminal member include: various rubber materials such as butyl rubber, halogenated butyl rubber, and ethylene propylene rubber; thermoplastic resins such as polyethylene, polystyrene, polyvinyl chloride, polyester, and polycarbonate; and thermosetting resins such as phenolic resins, eopxy resins, polyurethane resins, and silicon resins.

Furthermore, various kinds of materials, such as graphite, black lead, carbon black, fine powder of glass-shaped carbon, and short fibers obtained by cutting carbon fibers can be used as non-metal filler used for the terminal member.

Furthermore, a compounding ratio of non-metal filler with respect to a polymer matrix can be appropriately determined depending upon the kinds of a polymer matrix and carbon to be used, in consideration of required mechanical characteristics, electrical characteristics, durability, and the like.

Various kinds of carbon fibers such as polyacrylonitrile carbon fibers, pitch carbon fibers, and rayon carbon fibers can be used as the carbon fibers to be used for the conductive sheet, as long as they have conductivity high enough for allowing a current to flow at a substantially uniform current density from the entire surface of the conductive sheet.

Carbon fiber paper obtained by forming carbon fibers in a mat shape or in a paper shape using a paper making technique can also be used as the conductive sheet.

Furthermore, in order to improve the elasticity and handleability of the conductive sheet, carbon fibers or carbon fiber paper impregnated with a polymer elastomer such as silicon rubber or thermoplastic polyurethane can also be used as the conductive sheet.

Furthermore, in this embodiment, the case of using a circular conductive sheet has been described. However, the conductive sheet in any shape such as a square or a polygon can be used.

Furthermore, the attachment of the terminal member to the conductive sheet in the electrode of the present invention is not limited to the method described in the above embodiment. Any method can be used as long as the terminal member and the conductive sheet are appropriately fixed to such a degree as not to cause a problem in terms of the use, and the conduction required therebetween is ensured.

Furthermore, in the above embodiment, the case where the terminal member is attached to the vicinity of the center of the conductive sheet has been described. However, the terminal member can be attached to any one of the positions including the end portions of the conductive sheet.

Furthermore, in the above embodiment, the case where the male fitting portion is provided at the terminal member of the electrode has been described. However, the terminal member of the electrode of the present invention can also be provided with a female fitting portion in place of the male fitting portion, and the connection to an appliance such as an iontophoresis device or a low-frequency treatment device can also be performed via a connector having a male fitting portion to be fitted with the female fitting portion.

In the above embodiment, the case where the electrode of the present invention is used for an iontophoresis device or alow-frequency treatment device has been described. The electrode of the present invention can be used as an electrode for any other appliance which allows a current to flow to a living body, such as an electrocardiograph or a cosmetic instrument.

## Claims

1. An electrode (10a, 10b, 10c, 10d, 10e, 10f), **characterized by** comprising:
a conductive terminal member (11) formed of a non-metal material; and
a conductive sheet (12) formed of a non-metal material and attached to the terminal member (11),
the conductive sheet (12) having a specific resistance lower than a specific resistance of the terminal member (11).

2. An electrode (10a, 10b, 10c, 10d, 10e, 10f) according to claim 1 , **characterized in that** the conductive sheet (12) has a surface resistance of 1 to 30 Ω/(square).

3. An electrode (10a, 10b, 10c, 10d, 10e, 10f) according to claim 1 , **characterized in that** the conductive sheet (12) has a surface resistance of 1 to 10 Ω/(square).

4. An electrode (10a, 10b, 10c, 10d, 10e, 10f) according to claim 1 , **characterized in that** the conductive sheet (12) is formed of one of carbon fibers and carbon fiber paper.

5. An electrode (10a, 10b, 10c, 10d, 10e, 10f) according to claim 1 , **characterized in that** the conductive sheet (12) is formed of one of carbon fibers and carbon fiber paper impregnated with a polymer elastomer.

6. An electrode (10a, 10b, 10c, 10d, 10e, 10f) according to any one of claims 1 to 5, **characterized in that** the terminal member (11) is formed of a polymer matrix and anon-metal conductive filler dispersed in the polymer matrix.

7. An electrode (10a, 10b, 10c, 10d, 10e, 10f) according to claim 6, **characterized in that** the non-metal filler comprises carbon.

8. An electrode (10a) according to claim 6 or 7 , **characterized in that** the terminal member (11) is attached to the conductive sheet (12) by being solidified under a condition that one of the carbon fibers and the carbon fiber paper are impregnated with a part of the polymer matrix.

9. An electrode (10a) according to claim 8, **characterized in that** the conductive sheet (12) is attached to the terminal member (11) by integral molding.

10. An electrode (10a, 10b, 10c, 10d, 10e, 10f) according to any one of claims 6 to 9, **characterized in that** the polymer matrix is silicon rubber.

11. An electrode (10a, 10b, 10c, 10d, 10e, 10f) according to any one of claims 1 to 10, **characterized in that** the terminal member (11) has a fitting portion to be fitted with a connector connected to a power source (23).

12. An electrode (10a, 10b, 10c, 10d, 10e, 10f) according to anyone of claims 1 to 11, **characterized in that** a metal reinforcing member (15) is attached to the terminal member (11).

13. An iontophoresis device (20a, 20b, 20c), comprising a power source (23), a working electrode structure (21), and a nonworking electrode structure (22), **characterized in that**:
at least one of the working electrode structure (21) and the nonworking electrode structure (22) includes an electrode (30, 40) having:
a conductive terminal member (11) formed of a non-metal material; and
a conductive sheet (12) formed of a non-metal material and attached to the terminal member (11), the conductive sheet (12) having a specific resistance lower than a specific resistance of the terminal member (11).

14. An iontophoresis device (20a, 20b, 20c), comprising a power source (23), a working electrode structure (21), and a nonworking electrode structure (22), **characterized in that**:
the working electrode structure (21) includes:
a first electrode (30) connected to a terminal of a first conductivity of the power source (23);
a first conductive medium layer (33) placed on a front side of the first electrode (30);
a first ion-exchange membrane (34) for selecting ions of a second conductivity that is opposite to the first conductivity, the first ion-exchange membrane (34) being placed on a front side of the first conductive medium layer (33);
a drug layer (35) for holding a drug solution containing a drug that is dissociated to ions of the first conductivity, the drug layer being placed on a front side of the first ion-exchange membrane (34); and
a second ion-exchange membrane (36) for selecting ions of the first conductivity, the second ion-exchange membrane (36) being placed on a front side of the drug layer (35); and
the nonworking electrode structure (22) includes:
a second electrode (40) connected to a terminal of the second conductivity of the power source (23); and
a second conductive medium layer (43) placed on a front side of the second electrode (40); and
at least one of the first electrode (30) and the second electrode (40) comprises:
a conductive terminal member (11) formed of a non-metal material; and
a conductive sheet (12) formed of a non-metal material and attached to the terminal member (11), the conductive sheet (12) having a specific resistance lower than a specific resistance of the terminal member (11).

15. An iontophoresis device (20a, 20b) according to claim 14, **characterized in that** the nonworking electrode structure (22) further includes a third ion-exchange membrane (46) for selecting ions of the second conductivity, the third ion-exchange membrane (46) being placed on a front side of the second conductive medium layer (43).

16. An iontophoresis device (20a) according to claim 14, **characterized in that** the nonworking electrode structure (22) further includes:
a fourth ion-exchange membrane (44) for selecting ions of the first conductivity, the fourth ion-exchange membrane (44) being placed on a front side of the second conductive medium layer (43);
a third conductive medium layer (45) placed on a front side of the fourth ion-exchange membrane (44); and
a fifth ion-exchange membrane (46) for selecting ions of the second conductivity, the fifth ion-exchange membrane (46) being placed on a front side of the third conductive medium layer (45).
